# EUROPÄISCHE PATENTSCHRIFT

(11) **EP 0 637 343 B1**
(45) Veröffentlichungstag und Bekanntmachung des Hinweises auf die Patenterteilung: **14.07.1999**
(21) Anmeldenummer: 93911461.7
(22) Anmeldetag: 13.04.1993
(51) Int. Cl.: C12S 13/00, C02F 3/34, B09B 3/00, C12N 1/20, C12N 1/14

(54) **GEMISCH VON MIKROORGANISMEN, DESSEN VERWENDUNG ZUR BIODEGRADATION VON KOHLENWASSERSTOFFEN, SOWIE VERFAHREN ZU SEINER APPLIKATION**
MIXTURE OF MICROORGANISMS, THE USE OF THE MIXTURE IN THE BIODEGRADATION OF HYDROCARBONS, AND A METHOD OF APPLYING THE MIXTURE
MELANGE DE MICRO-ORGANISMES, SON UTILISATION POUR LA BIODEGRADATION D'HYDROCARBURES, ET PROCEDE POUR SON APPLICATION

(30) Priorität: 15.04.1992 CZ 115792
(43) Veröffentlichungstag der Anmeldung: 08.02.1995
(73) Patentinhaber: Biorem AG, 6340 Baar (CH)
(72) Erfinder: HORAKOVA, Dana, 630 00 Brno (CZ); NEMEC, Miroslaw, 664 31 Ceska (CZ)
(74) Vertreter: Felber, Josef
(86) Internationale Anmeldenummer: EP9300897
(87) Internationale Veröffentlichungsnummer: WO9321348

(56) Entgegenhaltungen:
- EP-A- 270 805
- EP-A- 289 350
- US-A- 4 415 661
- US-A- 4 535 061
- US-A- 4 593 003

## Beschreibung

Die Erfindung betrifft ein Gemisch von einzelnen, in der Natur vorkommenden Mikroorganismen, das in seiner Zusammensetzung Kohlenwasserstoffe biologisch degradieren kann. Bei den Kohlenwasserstoffen ist dabei besonders an Erdöl, seine verschiedenen Derivate, sowie an Erdölprodukte gedacht. Im weiteren bezieht sich die Erfindung auch auf die Verwendung des Gemisches zur Biodegradation von Kohlenwasserstoffen. Schliesslich gibt die Erfindung spezielle Verfahren zur Kultivierung und Applikation des Gemisches für die Biodegradation von ölverschmutzten Böden und Gewässern an.

Das Interesse an der gezielten Nutzung von Mikroorganismen für die biologische Degradation von Erdöl, seiner Derivate und produkte hat in den letzten Jahren stark zugenommen. Schon im Jahre 1903 wurden die ersten Organismen beschrieben, welche Kohlenwasserstoffe als Kohlenstoff- und Energiequelle nutzten. Gegenwärtig sind mehr als 40 Mikroorganismen-Gattungen bekannt, welche die aliphatischen oder aromatischen Kohlenwasserstoffe im Zuge einer biologischen Degradation nutzen. Diese Organismen wurden aus den Ozeanen, aus Süsswasser-Gewässern sowie aus dem Boden isoliert.

Die Anzahl der Bakterien und der Pilze, die fähig sind, Erdöl-Kohlenwasserstoffe biologisch zu degradieren, steigt nach Erdölhaverien lokal steil an. Diese Stoffe treten in bedeutendem Mass als Selektionsgas auf.

Vom genetischen Gesichtspunkt her gesehen ist es einsehbar, dass die Fähigkeit eines Grossteils von Mikroorganismen, die Erdölkohlenwasserstoffe zu degradieren, von Plasmiden kontrolliert wird. Durch den Nachweis der Uebertragung von Plasmiden wurde es auch möglich, den steilen Anstieg der Anzahl degradierender Organismen zu erklären.

Das Erdöl als Substrat für Mikroorganismen stellt ein extrem komplexes Gemisch von Kohlenwasserstoffen dar. Die saturierte Erdölfraktion umfasst n-Alkane, verzweigte Alkane und Zykloalkane. Die Biodegradation der n-Alkane bis C₄₄ wird am häufigsten von der monoterminalen Attacke unter Entstehung von Karbonsäuren verwirklicht, die weiterhin durch β-Oxidation abgebaut werden. Dieser Prozess kann jedoch eine Akkumulierung mancher mehr oder weniger toxischer Fettsäuren herbeiführen.

Die verzweigten isoprenoiden Alkane werden durch w-Oxidation unter Entstehung von Dikarbonsäuren abgebaut. Gegen eine Biodegradation sind die terminal verzweigten Alkane sehr widerstandsfähig. Sie blockieren die β-Oxidation und haben die Tendenz, sich im Milieu zu kumulieren. Nach der spontanen Degradation einer Erdölverunreinigung kommt es häufig zum Entstehen tripentazyklischer Verbindungen, die im Milieu persistent sind. Daraus geht hervor, dass die qualitative Zusammensetzung des Erdöls und der Erdölprodukte ihre Degradabilität bedeutend beeinflusst.

Bei dem Metabolismus der Erdölkohlenwasserstoffe spielen zwei weitere Prozesse eine bedeutsame Rolle - die Kooxidation und Prolongation. Manche Erdölkomponenten, die nach etablierter Meinung der Fachwelt nicht abbaubar sind, können nämlich von Mikroorganismen in Gegenwart anderer Kohlenwasserstoffe, welche das Wachstum von Mikroorganismen ermöglichen, degradiert werden. Der Degradierungsprozess des erfindungsgemässen Gemisches von Kohlenwasserstoffen, bedingt durch Kooxidationsprozesse, ist deshalb keinesfalls vergleichbar mit der Fähigkeit der Reinkulturen, die einzelnen Kohlenwasserstoffe zu degradieren.

Die Geschwindigkeit einer mikrobialen Degradation der Erdölkohlenwasserstoffe wird von einer ganzen Reihe von Faktoren beeinflusst, welche direkt oder indirekt auf das Wachstum und den Metabolismus der mikrobialen Population einwirken. Als bedeutsam zeigt sich der physikalische Zustand der Kohlenwasserstoffe, weil die Kohlenwasserstoffe an der "Trennlinie" Wasser-Kohlenwasserstoff am intensivsten degradiert werden. Zu den weiteren abiotischen Faktoren gehören die Temperatur und die zur Verfügung stehenden Nährstoffe. Die meisten heterotrophen Mikroorganismen sind fähig, Kohlenwasserstoffe in einem breiten Temperaturbereich von 0°C bis 70°C zu degradieren. Die Temperatur beeinflusst nicht nur die Geschwindigkeit, mit welcher die Degradation verläuft, sondern vor allem auch den physikalischen Zustand der Kohlenwasserstcffe, was in enger Verbindung zum selektiven Druck der Temperatur auf die mikrobialen Gemeinschaften steht, die das Oekosystem bilden. Bei der komplexen Bewertung muss die Temperatur zusammen mit anderen Faktoren berücksichtigt werden, jedoch muss sie keineswegs als limitierender Faktor auftreten.

Die Geschwindigkeit der Degradation von Erdölprodukten wird in bedeutendem Ausmass von der Anwesenheit von Stickstoff und und Phosphor beeinflusst, welche gemeinsam mit kohlenstoffhaltigem Spaltstoffen in die Biomasse eingebaut sind. Die optimalen Mengenverhältnisse zwischen dem Kohlenstoff und dem Stickstoff einerseits und dem Kohlenstoff und dem Phosptor andrerseits hängen von der qualitativen Zusammensetzung des Erdöls oder der Erdölprodukte und den abiotischen Faktoren des Milieus ab. Stickstoff und Phosphor sind auch die üblichen Komponenten der "Stimulatoren", welche die Biodegradationsprozesse stimulieren.

Für die gewerbliche Anwendbarkeit von Mikroorganismen im Zuge der Sanierung von kontaminierten Böden oder Gewässern spielt die Abbaurate sowie die Qualität der Degradation eine herausragende Rolle. Um diese Abbaurate zu beschleunigen, unterscheidet man nach drei Hauptkategorien:
1. Fertilisation: Hierbei beschränkt man sich darauf, das Milieu für die im kontaminierten Boden oder Gewässer inherenten Mikroorgansimen zu optimieren. Man geht davon aus, dass in einem solchen verschmutzten Boden oder Gewässer öldegradierende Mikroorganismen ohnehin in ausreichender Qualität und Quanität vorhanden sind, und konzentriert sich deshalb darauf, hauptsächlich Stickstoff, Phosphor und Kalium sowie gegebenenfalls einige Spurenelemente zuzugeben, sowie die Atmungsfähigkeit der vorhandenen, stets aeroben Mikroorganismen durch Zufuhr von Luft zu unterstützen. Hierfür kann der Boden regelmässig gelockert und gewendet werden, oder aber mittels Luftleitungen und Pumpen mit Luft versorgt werden. Oftmals wird ein mehrere Meter hoch aufgeschichteter Erdhaufen über ein System von durch ihn verlegten perforierten Rohren besaugt und bepumpt (Vakuum-Heap-System). Die Fachwelt verspricht sich von dieser Technik die grössten Erfolge, weil man insbesondere bei Ölunfällen in Gewässern davon ausgeht, dass dort öldegradierende Mikroorganismen rasch in ausreichender Menge vorhanden sein werden, und die Degradation eher infolge der mangelnden Versorgung dieser Organismen mit Nahrung und Luft gehemmt wird.
2. Seeding: Als eigenständige Technik oder zusätzlich zu obengenannter Methode wird das Seeding als Technik angewendet. Darunter versteht man das Einbringen von zusätzlichen, öldegradierenden Mikroorganismen in das kontaminierte Material. Solche Präparate, die von verschiedenen Firmen in Umgebungen mit Erdölverschmutzungen eingesetzt werden, enthalten üblicherweise Organismen einer einzigen Art. Der Einsatz solcher Präparate ist für frische Verunreinigungen dort angebracht, wo im Milieu noch nicht Produkte des Metabolismus zugegen sind, welcher infolge der dort natürlicherweise vorhandenen Organismen abläuft. In der Fachwelt hat sich aber die Meinung etabliert, dass mit der zusätzlichen Einführung weiterer natürlicher Mikroorganismen nicht sehr viel zu gewinnen ist. Darüberhinaus neigt die Oeffentlichkeit im allgemeinen zu Vorbehalten gegenüber der Transplantation von fremden Mikroorganismen, weil - zurecht oder zu unrecht -, deren unkonrollierbares Wachstum, begleitet von unliebsamen, allenfalls toxischen Nebenwirkungen, befürchtet wird.
3. GEM: Dieses ist eine Abkürzung für die englische Bezeichnung "Genetically Engineered Microorganisms", also genetisch manipulierte Mikroorganismen, welche besonders herausragende Qualitäten in bezug auf ihre Öl-Degradierungsfähigkeit aufweisen. Gegenüber dieser Technik bestehen die grössten Vorbehalte, sodass deren Anwendung wohl zunehmend beschränkt wird.

Die Aufgabe der vorliegenden Erfindung ist es, ein Gemisch von natürlichen Mikroorganismen anzugeben, welches eine bedeutend höhere Degradationsrate für Kohlenwasserstoffe in Böden und Gewässern aufweist sowie leicht kultivierbar und praktisch anwendbar ist. Weiter ist es eine Aufgabe der Erfindung, anzugeben, wie dieses Gemisch verwendet wird, sowie ein Verfahren zur Kultivierung und Applikation anzugeben.

Diese Aufgabe wird gelöst von einem Gemisch von natürlichen Mikroorganismen für die Biodegradation von Kohlenwasserstoffen, insbesondere von Erdöl und Erdölprodukten, das sich dadurch auszeichnet, dass es besondere Stämme von **Pseudomonas putida** und **Geotrichum candidum** im Verhältnis der Zellenzahl von 5 : 1 bis 1 : 1 enthält. Diese speziell aus der Natur isolierten Stämme von Pseudomonas putida und Geotrichum candidum sind bei der Tschechischen Hinterlegungsstelle für Mikroorganismen (CCM - Czech Collection of Microorgamisms) Tvrdého 14, CR-602 00 Brno, hinterlegt worden. Der Modellstamm Pseudomonas putida wurde am 3. Juli 1992 unter der Nummer CCM 4307 mit der Bezeichnung *Pseudomonas sp. RC1* hinterlegt, wobei als Datum für die internationale Hinterlegung der 31. August 1992 gilt. Der Modellstamm Geotrichum candidum wurde am 12. März 1993 unter der Nummer 8170 mit der Bezeichnung *Geotrichum candidum RC1* hinterlegt, was auch dem Datum der internationalen Hinterlegung entspricht. Unter den gleichen Nummern wurden diesen Mikroorganismen am 30. März 1993 Lebensfähigkeitszeugnisse von der nämlichen internationalen Hinterlegungsstelle ausgestellt.

Für die Verwendung des Gemisches aus diesen hinterlegten Mikroorganismen im Zuge der Sanierung von kontaminierten Böden und Gewässern wird es bei einem pH-Wert im Bereich zwischen 4,5 bis 7,5 und im Temperaturbereich von 5° bis 35°C in einer Menge von zwischen 1 bis 10 Gramm pro Liter appliziertes Anwendungspräparat eingesetzt. Pro m² kontaminierter Boden werden etwa 5 bis 20 Liter Anwendungspräparat ein- oder mehrmals ausgebracht.

Das Verfahren zu seiner Verwendung besteht grundsätzlich im ein- oder mehrmaligen Besprühen der kontaminierten Masse mit in Wasser gelöstem Gemisch, wonach das Milieu in der kontaminierten Massen durch Zufuhr von Luft und Nährlösung nach der herkömmlichen Methode optimiert wird.

Die Erfindung geht grundsätzlich von der Erkenntnis aus, dass es entgegen der verbreiteten Ansicht von Fachleuten möglich ist, die Degradationsgeschwindigkeit von Erdölstoffen, wie sie durch die lokal natürlich vorhandenen Mikroorgansimen-Gemeinschaften in einem bereits optimierten Milieu gegeben ist, durch das Inkorporieren von künstlich gezüchteten Gemeinschaften natürlicher Mikroorganismen, welche eine erhöhte Affinität zu den gegebenen Stoffen besitzen, entscheidend zu erhöhen.

Als sehr vorteilhaft erweist sich dabei die Applikation künstlich zusammengestellter und gezüchteter Gemeinschaften natürlicher Mikroorganismen, welche die Fähigkeit besitzen, in grösserem Ausmass Koordinationsprozesse zu nutzen und dadurch die Degradationsprozesse bedeutend beschleunigen und "qualitätsfördernd" auf sie wirken.

Das erfindungsgemässe Gemisch ermöglicht eine effektivere Nutzung des Substrates der Erdölstoffe durch eine Vereinigung bzw, ein Gemisch (Konsortium) von natürlichen Mikroorganismen. Das Wesentliche der Erfindung bildet ein Gemisch von Mikroorganismen, das die Mikroorganismen **Pseudomonas putida** und **Geotrichum candidum** im Verhältnis der Zellenzahl 5:1 bis 1:1 enthält. Beide Organismen wurden aus der Umgebung der Erdölbohrung Hodonin in der Tschechischen Republik isoliert und sind unter den internationalen Hinterlegungsnummern CCM 4 307, bzw. CC M 8170 hinterlegt. Nach der Isolierung wurden die Mikroorganismen nicht mehr genetisch aufbereitet, sondern natürlich belassen.

Pseudomonas putida ist ein gebräuchliche Kontaminat des Bodens, des Wassers und der Pflanzen. Es kommt auch in der Umgebung von Krankenhäusern vor. Selten wird es aus klinischen Proben als opportun pathogen isoliert.
Geotrichum candidum andrerseits ist ein übliches Kontaminat von Milch sowie von verschmutztem Wasser. Selten kommt es im klinischen Material als bedingt pathogen für Mensch und Tier vor.
Auch das Gemisch selbst ist weder toxisch noch pathologisch.
Dieses wurde anhand umfangreicher wissenschaftlicher Tests nachgewiesen und ist zertifiziert.
Unter anderem wurde das Gemisch, nachfolgend stets BIOREM RC 1 genannt, in Flüssigform an 24 Kaninchen, 60 Mäuse und 30 Meerschweinchen in je 6 Gruppen im Rahmen eines Langzeitversuches teils intravenös injiziert, teils verfüttert. Dabei wurde folgendes wissenschaftlich nachgewiesen:
1. Es kam weder zu Gewichtsabnahmen noch zu akuten oder chronischen Erkrankungen irgendwelcher Art bei den behandelten Tieren.
2. Es wurde keine Veränderung im Benehmen der Tiere beobachtet wie zum Beispiel Appetitverlust, Gereiztheit oder Apathie.
3. In jeder Gruppe wurden makroskopische Veränderungen auf gewissen Organen untersucht, nämlich der Nieren, der Leber, der Lunge, der Lymphknoten und des Herzens. Es wurden keine pathologische oder toxikologische Veränderungen gefunden.

Die Art und Weise der Biodegradation von Kohlenwasserstoffen durch Verwendung dieses Gemisches der Mikroorganismen Pseudomonas putida und Geotrichum candidum beginnt damit, dass dieses Gemisch extern vermehrt wird und dass man es auf einem synthetischen Medium in Gegenwart von Ölsäure induziert. Hierauf wirkt man damit unter Zufuhr von Sauerstoff auf die zu degradierenden Kohlenwasserstoffe ein, in Gegenwart von Stimulationsstoffen, insbesondere von Stickstoff und Phosphor, eventuell von Spurenelementen, wobei das Verhältnis des Kohlenstoffes und des Stickstoffes während der Wirkung des Gemisches der vorangehend genannten Mikroorganismen im Bereich der Gewichtsverhältnisse 10 bis 100 : 1 erhalten wird.

Das optimale Milieu für eine Biodegradation der Kohlenwasserstoffe durch dieses Gemisch von Mikroorganismen ist der Erfindung nach ein Milieu mit einem pH von 4,5 bis 7,5 und einer Temperatur von 5° bis 35°C.

Für die Aufbereitung und Indikation des Gemisches von Mikroorganismen ist es nach der Erfindung angebracht, ein synthetisches Medium zu benutzen, das nur Phosphor und Stickstoff in löslicher Form, sowie Ölsäure enthält. Es ist vorteilhaft, den Ausgangs-pH-Wert des Kultivationsmediums im Bereich von 4,5 bis 7,5 zu wählen. Unter intensivem Durchlüften bei einer Temperatur von 20°C - 25°C beträgt die Kultivationszeit 4 - 10 Tage, je nach den an die Dichte der bakterialen Suspension gestellten Anforderungen. Das Präparat kann dann einer Lyophilisierung oder einer Trocknung unterzogen werden oder man kann es nach Wasserentzug in einem Gel verankern.

Das Präparat kann man nach der Methode "ex situ" oder "in situ" durch Aufspritzen auf kontaminierten Boden benützen oder durch Inokulieren in eine kontaminierte Wasserquelle, eventuell in durch Erdölstoffe kontaminiertes Wasser unter gleichzeitigem Hinzufügen eines metabolischen Stimulators. Die Methode bietet auch die Möglichkeit, einen mit synthetischem Milieu, einem Stimulator und kontaminierten Wasser gefüllten Fermentor zu nutzen, Das mikrobiale Gemisch wird in der Menge hinzugefügt, die der optischen Densität bei 620 nm (OD₆₂₀) im Bereich von 0,60 bis 1,90 entspricht. Der pH-Wert des Milieus wird in den Grenzen pH = 4,5 bis 7,5 gehalten. Der Degradationsprozess verläuft unter aeroben Bedingungen.

Insofern das mikrobiale Gemisch durch Aufspritzen auf den Boden nach der Methode "ex situ" appliziert wird, benutzt man 1 bis 10 Liter der gemischten Kultur mit dem Wert der optischen Densität bei 620 nm im Bereich 0,60 bis 1,00 pro 1 m³ Erdreich, in Abhängigkeit von der Feuchte und der Bodenstruktur.

Bei der Methode "in situ" wendet man 5 bis 15 Liter dieser Kultur pro m² an. In beiden Fällen ist es notwendig, das Erdreich vor und auch nach dem eigentlichen Applizieren zu lockern, um damit eine geeignete Aeration zu sichern. Die gemischte Kultur enthält bei der Anwendung zugleich Stimulationsstoffe.

Das Ergebnis der Anwendung des erfindungsgemässen Gemisches natürlicher Mikroorganismen ist eine Degradation des überwiegenden Teiles der in der Umgebung vorhandenen Erdölstoffe, die einerseits in Kohlendioxid (CO₂) übergeführt, anderseits in das Zellmaterial eingebaut werden. Gegebenenfalls können die Metaboliten in das aussenstehende Milieu ausgeschieden werden.
Der Vorteil dieses Gemisches und seiner Anwendung beruht vor allem darin, dass die entstehenden Metaboliten nicht toxisch sind. Weiter ist bei der Anwendung des Gemisches von Mikroorganismen auch die hohe Anfangsgeschwindigkeit der Degradation kontaminierender Stoffe von grosser Bedeutung. Ein weiterer Vorteil besteht darin, dass durch die Anwendung des Präparates die Anzahl der Mikroorganismen im Milieu in bedeutendem Mass angehoben wird, was insbesondere zur Verbesserung der Struktur des Erdreichs beiträgt und dessen Zurückführung in den fast ursprünglichen Zustand sehr förderlich ist. Angesichts dessen, dass die angewandten Organismen aus der natürlichen Umgebung isoliert und genetisch nicht manipuliert worden sind, besteht keine Gefahr eines negativen Einflusses auf die Biosphäre.

### Anwendungsbeispiele

### Beispiel 1:

1000 ml des synthetischen Mediums mit dem Anfangsvernältnis Kohlenstoff : Stickstoff Phosphor = 800 bis 400 : 6 : 1 wurden mit der vorbereiteten induzierten Kultur (BIOREM RC1) inokuliert, deren optische Densität OD₆₂₀ = 0,650 ist, wobei das Verhältnis der Zellenzahl Pseudomonas putida : Geotrichum candidum = 3:1 war. Der Ausgangswert betrug pH = 6,0. Die Kultivierung verlief bei 20°C bis 25°C unter Durchlüftung. Der Stimulationsstoff wurde zu Beginn der Kultivierung und ferner immer nach 5 bis 10 Tagen in solch einer Menge hinzugefügt, dass das Verhältnis zwischen dem Kohlenstoff und dem Stickstoff 60 bis 100 : 1 war. Der Gehalt an Erdölstoffen wurde mit Hilfe der Infrarot-Spektroskopie durch Vergleich der Transmittanz der Probe und des Standards bei einer Wellenzahl von 2920 cm⁻¹ gemessen.

| Probe | RC1 | Stimulator | Gehalt an Erdölstoffen (g.l⁻¹) | | | |
|---|---|---|---|---|---|---|
| | | | 0 Tage | 10 Tage | 24 Tage | 38 Tage |
| rohes Erdöl | | | | | | |
| | - | - | 18,3 | 18,3 | 18,4 | 18,2 |
| | + | - | 34,1 | 22,1 | 15,0 | 6,1 |
| | + | + | 34,0 | 18,2 | 11,7 | 1,2 |
| | | | | | | |
| gebrauchtes Motorenöl | | | | | | |
| | + | + | 18,0 | 7,8 | 1,6 | 0,23 |
| | | | | | | |
| gebrauchtes Motorenöl und Rohöl im Verhältnis 1:1 | | | | | | |
| | + | + | 18,3 | 8,2 | 3,0 | 0,62 |

### Beispiel 2:

1000 ml des synthetischen Mediums wurden mit der vorbereiteten induzierten Kultur (BIOREM RC1) derart inokuliert, dass OD₆₂₀ = 0,700 war, bei einem Verhältnis der Zellenzahl Pseudomonas : Geotrichum = 3 : 1. Die getesteten Erdölprodukte wurden dem Gemisch zugefügt bis zur Konzentration 10g.1⁻¹. Die Kultivierung verlief bei einer Temperatur von 20°C bis 25°C unter Durchlüftung. Der Stimulationsstoff wurde immer nach 7 Tagen hinzugefügt - nach denselben Bedingungen wie in Beispiel 1. Der Gehalt an Erdölstoffen wurde mit Hilfe von Infrarot-Spektroskopie festgestellt. Es wurde die Transmittanz der Probe und des Standards bei einer Wellenzahl von 2920 cm⁻¹ gemessen.

| Probe Degradationsdauer | Gehalt an Erdölstoffen in Gramm pro Liter | | | |
|---|---|---|---|---|
| | 10 Tage | 24 Tage | 38 Tage | 42 Tage |
| Petroleum RT | 3,58 | 0,34 | 0,21 | 0,21 |
| Motorenöl | 6,63 | 2,09 | 0,35 | 0,21 |
| MS 8P OLEMS-2 | 4,25 | 1,54 | 0,52 | 0,20 |
| Hydraulikflüssigkeit AMG 10 | 2,12 | 0,80 | 0,24 | 0,20 |
| Benzin B3V | 3,63 | 2,07 | 0,80 | 0,26 |
| Petroleum PN-3 | 2,94 | 1,80 | 0,25 | 0,22 |
| Petroleum PN-3 mit Antioxidans | 2,86 | 1,92 | 0,32 | 0,25 |

### Beispiel 3:

Auf 1000 g Erdreich, das nach einer Havarie durch Erdölprodukte kontaminiert war, wurden 1000 ml der gemischten induzierten Kultur BIOREM RC 1 bei OD₆₂₀ = 0,600 angewandt, bei einem Verhältnis der Zellenanzahl Pseudomonas : Geotrichum = 1 : 1. Die Kultivierung verlief bei 20°C bis 25°C in Glaswannen. Jeden zweiten Tag wurde das Erdreich durch mehrfaches Umwenden durchlüftet. Der Stimulationsstoff wurde immer nach 8 bis 10 Tagen unter den in Beispiel 1 angeführten Bedingungen hinzugefügt. Der Gehalt an Erdölstoffen wurde nach der Extraktion Tetrachlor mittels Infrarot-Spektroskopie bei einer Wellenzahl von 2920 cm⁻¹ gemessen.

| Probe Degradationsdauer | Gehalt an Erdölstoffen in Gramm pro Liter | | |
|---|---|---|---|
| | 10 Tage | 24 Tage | 40 Tage |
| ursprüngliche Probe | 1,2 | 1,2 | 1,2 |
| Erdreich + RC1 | 0,61 | 0,57 | 0,09 |
| Erdreich + Kompost + RC1 | 0,41 | 0,32 | 0,08 |

Die Kontaminierung des Erdreiches war verhältnismässig niedrig und demzufolge wurde die gemischte Kultur BIOREM RC1 nur zu Beginn des Biodegradationsprozesses angewandt. Im Falle einer grösseren Verunreinigung kann man den Prozess durch wiederholte Beigabe der gemischten Kultur im Verlauf der Biodegradierung beschleunigen.

### Beispiel 4:

Eine Menge von 1'000 ml synthetischen Mediums wurde mit Öl in einer Konzentration von 10'000 mg pro Liter verschmutzt. Bei der Degradation wurde das Verhältnis von zugefügtem Stickstoff und noch vorhandenem Kohlenstoff in bestimmten Grenzen gehalten. Dieser Versuch wurde mit verschiedenen Ölderivaten durchgeführt und es wurden folgende Degradationsraten erzielt:

| Probenmaterial Degradationsdauer: | Masse von Ölderivaten in mg 1⁻¹ | | | |
|---|---|---|---|---|
| | 10 Tage | 20 Tage | 30 Tage | 40 Tage |
| Rohöl | 4'920 | 2'420 | 1'080 | 107 |
| Motorenöl | 4'620 | 1'870 | 280 | 115 |
| Altes Motorenöl | 5'420 | 2'640 | 1'430 | 360 |
| Petroleum | 3'010 | 1'970 | 490 | 195 |
| Benzin | 3'980 | 2'140 | 910 | 210 |

### Beispiel 5:

Eine Menge von 1 kg trockenem Erdreich, verschmutzt mit 20'000 mg einer Mischung Rohöl, verschmutztem Öl und Petroleum wurde mit 20 Gramm BIOREM RC 1 behandelt. Es wurden folgende Degradationsraten erzielt:

| Probenmaterial Degradationsdauer: | Masse der Öl-Derivate in mg pro kg trockenes Erdreich | | |
|---|---|---|---|
| | 10 Tage | 30 Tage | 40 Tage |
| Rohöl | 13'200 (66,0%) | 7'120 (35,6%) | 890 (4,45%) |
| | | | |
| Motorenöl | 11'610 (58,05%) | 4'560 (22,8%) | 410 (2,05%) |
| | | | |
| Petroleum | 9'980 (49,9%) | 3'120 (15,6%) | 310 (1,55%) |

In Klammern ist der jeweils zu diesem Zeitpunkt verbleibende prozentsatz des Öl-Derivates im Vergleich zum Beginn (100%) angegeben.

### Beispiel 6:

22 m³ öl-kontaminiertes Erdreich wurde mit Kompost vermischt in einer Schicht von 30 cm Höhe im Freien aufgetragen. Alle drei Tage wurde das Erdreich gelockert und gewendet. Eine erste Kontrolle von gleichermassen kontaminiertem Erdreich (Nr. 1) wurde bloss mit Kompost gemischt, aber nicht mit BIOREM RC 1 behandelt. Eine zweite Kontrolle von gleichermassen kontaminiertem Erdreich (Nr. 2) wurde überhaupt nicht behandelt. Die folgenden Resultate wurden erzielt:

| Probenmaterial Degradationsdauer | Konzentration von Öl-Derivaten in mg pro kg trockenes Erdreich | | | |
|---|---|---|---|---|
| | 0 Tage | 15 Tage | 35 Tage | 60 Tage |
| Erdreich mit Kompost und BIROEM RC1 | 1'698 (100,0%) | 267 (15,72%) | 151 (8,89%) | 98 (5.77%) |
| | | | | |
| Kontrolle Nr. 1 | 1'698 (100,0%) | 1'176 (69,26%) | 912 (53,71%) | 657 (38,7%) |
| | | | | |
| Kontrolle Nr. 2 | 1'698 (100,0%) | 1'656 (97,53%) | 1'589 (93,58%) | 1'504 (88,6%) |

in Klammern ist der jeweils zu diesem Zeitpunkt verbleibende Prozentsatz des Öl-Derivates im Vergleich zum Beginn (100%) angegeben.

### Beispiel 7:

Ölstoff-Biodegradation in Schwermetall-belastetem Schlamm

| Konzentration der Öl-Derivate im Schlamm (mg pro Liter) : | | | | |
|---|---|---|---|---|
| Degradationsdauer: | 3 Tage | 14 Tage | 21 Tage | 35 Tage |
| | 78'600 ± 170 | 91'500 ± 130 | 29'400 ± 140 | 9'300 ± 90 |

### Beispiel 8:

Ölstoff-Biodegradation des Abfallwassers auf dem festen Träger (Testperiode: 11. Juli 1992 bis 11. Oktober 1992):

| Konzentration der Öl-Derivate im Wasser (g pro kg) | | | | |
|---|---|---|---|---|
| Degradationsdauer: | 0 Tage | 30 Tage | 60 Tage | 90 Tage |
| | 84,39 | 64,50 | 38,22 | 8,64 |

### Beispiel 9:

Motorenöl-Biodegradation im Boden (anlässlich eines Ölunfalls) :

| Methode Degradationsdauer | Konzentration der Öl-Derivate in mg/kg Boden | | | | |
|---|---|---|---|---|---|
| | 0 Tage | 10 Tage | 20 Tage | 50 Tage | 90 Tage |
| Kompost | 813 | 564 | 380 | 309 | 217 |
| BIOREM RC 1 | 813 | 407 | 317 | 185 | 119 |
| Kompost und BIOREM RC 1 | 813 | 413 | 254 | 137 | 91 |
| Kontrolle | 813 | | | | 691 |

### Beispiel 10:

Ölstoff-Biodegradation in einem Boden mit alter Verschmutzung (Testperiode: 13, Juli 1992 bis 19. September 1992):

| Konzentration der Öl-Derivate in mg/kg Boden | | | | |
|---|---|---|---|---|
| Degradationsdauer: | 0 Tage | 16 Tage | 36 Tage | 67 Tage |
| | 1'626 | 280 | 164 | 128 |

### Beispiel 11:

Transformatorenöl-Biodegradation im Boden anlässlich eines Ölunfalls (Testperiode: 31. August 1992 bis 26. Oktober 1992):

| Methode Degradationsdauer | Konzentration der Öl-Derivate in mg/kg Boden | | | |
|---|---|---|---|---|
| | 0 Tage | 28 Tage | 45 Tage | 56 Tage |
| | 16'180 | 5'434 | 2'562 | 781 |

Als Vergleichswert zu diesen Beispielen sei hier erwähnt, dass man zur gewerblichen Degradation eines Bodens mit 2000 mg Kohlenwasserstoffen pro kg Trockensubstanz hinunter auf 282 mg pro kg Trockensubstanz ca. 30 Wochen durchaus als Richtwert betrachtet (Zeitschrift TR Technische Rundschau, Das Schweizer Industriemagazin, Heft 3, 1993, Seite 28).

Das künstlich zubereitete Gemisch von Mikroorganismen ist in natürlichen Bedingungen stabil. Es ist im weiten Bereich des pH-Wertes zwischen 4,5 bis 7,5 und im Temperaturbereich von 5° bis 35°C aktiv. Bedingung für das Erzielen einer maximalen physiologischen Aktivität ist eine optimale Sauerstoffzufuhr (für die Oxidation von 3,5 g rohen Erdöls wird minimal 1 g O₂ benötigt) und die Anwesenheit eines Stimulationsstoffes. Das Gemisch ist in der Lage, die Erdölkohlenwasserstoffe sowohl bei hoher Belastung (über 10 g/l) als auch bei relativ niedriger Belastung (um lg/1) zu degradieren. In beiden Fällen können nach durchgeführter Degradation die Konzentrationen der per Norm bewilligten Erdölstoffe detektiert werden.

Das Gemisch von Mikroorganismen ist grundsätzlich in der Lage, Kohlenwasserstoffe enthaltende Abfälle zu entsorgen, insbesondere Erdöl und seine Produkte, sowie Folgen einer ökologischen Katastrophe zu beseitigen, bei der es zur Kontamination des Bodens oder des Wassers durch Erdölstoffe gekommen ist.

## Patentansprüche

1. Gemisch von natürlichen Mikroorganismen für die Biodegradation von Kohlenwasserstoffen, insbesondere von Erdöl und Erdölprodukten, dadurch gekennzeichnet, dass es Pseudomonas putida gemäss der internationalen Hinterlegungsnummer Nummer CCM 4307 mit der Bezeichnung *Pseudomonas sp. RC1,* sowie Geotrichum candidum gemäss der internationalen Hinterlegung Nummer CCM 8170 mit der Bezeichnung *Geotrichum candidum RC1* im Verhältnis der Zellenzahl von 5 : 1 bis 1 : 1 enthält.

2. Gemisch nach Anspruch 1, dadurch gekennzeichnet, dass es Pseudomonas putida und Geotrichum candidum im Verhältnis der Zellenzahl von 1 : 1 enthält.

3. Gemisch nach Anspruch 1, dadurch gekennzeichnet, dass es Pseudomonas putida und Geotrichum candidum im Verhältnis der Zellenzahl von 2 : 1 enthält.

4. Gemisch nach Anspruch 1, dadurch gekennzeichnet, dass es Pseudomonas putida und Geotrichum candidum im Verhältnis der Zellenzahl von 3 : 1 enthält.

5. Gemisch nach Anspruch 1, dadurch gekennzeichnet, dass es Pseudomonas putida und Geotrichum candidum im Verhältnis der Zellenzahl von 4 : 1 enthält.

6. Gemisch nach Anspruch 1, dadurch gekennzeichnet, dass es Pseudomonas putida und Geotrichum candidum im Verhältnis der Zellenzahl von 5 : 1 enthält.

7. Verfahren zur Kultivierung des Gemisches nach einem der Ansprüche 1 bis 6, dadurch gekennzeichnet, dass das Gemisch induziert wird durch Kultivierung in Gegenwart von Ölsäure, unter Zufuhr von Sauerstoff und gegebenenfalls unter Zugabe von Stimulationsstoffen, insbesondere von Stickstoff und Phosphor, sowie gegebenenfalls von Spurenelementen.

8. Verfahren zur Applikation des Gemisches nach einem der Ansprüche 1 bis 6, dadurch gekennzeichnet, dass ein oder mehrmals zwischen 1 Gramm und 10 Gramm Gemisch pro Liter Anwendungspräparat in einer Dosierung des Anwendungspräparates je nach Grad der Bodenverschmutzung von 5 bis 20 Litern pro zu behandelndem m³ Boden appliziert wird, und dass Sauerstoff sowie Stimulationsstoffe, nämlich Stickstoff, Phosphor sowie Spurenelemente in optimaler Menge zugeführt werden, wobei das Verhältnis des Kohlenstoffes und des Stickstoffes während der Wirkung des Gemisches im Bereich 10 : 1 bis 100 : 1 gehalten wird.

9. Verfahren nach Anspruch 8, dadurch gekennzeichnet, dass das Milieu auf einem pH-Wert zwischen 4,5 bis 7,5 und auf einer Temperatur von 5° bis 35°C gehalten wird.

10. Verwendung eines Gemisches nach einem der Ansprüche 1 bis 6 zur gewerblichen Degradation von Kohlenwasserstoffen in Böden und Gewässern.

## Revendications

1. Mélange de micro-organismes naturels pour la biodégradation d'hydrocarbures, en particulier de pétrole et de ses produits, caractérisé en ce qu'il contient de la Pseudomonas putida, suivant le numéro de dépôt international CCM 4307, ayant la dénomination *Pseudomonas sp. RC1,* ainsi que du Geotrichum candidum, ayant la dénomination *Geotrichum candidum RC1,* le rapport du nombre de cellules étant de 5/1 à 1/1.

2. Mélange suivant la revendication 1, caractérisé en ce qu'il contient de la Pseudomonas putida et du Geotrichum candidum, le rapport du nombre de cellules étant de 1/1.

3. Mélange suivant la revendication 1, caractérisé en ce qu'il contient de la Pseudomonas putida et du Geotrichum candidum, le rapport du nombre de cellules étant de 2/1.

4. Mélange suivant la revendication 1, caractérisé en ce qu'il contient de la Pseudomonas putida et du Geotrichum candidum, le rapport du nombre de cellules étant de 3/1.

5. Mélange suivant la revendication 1, caractérisé en ce qu'il contient de la Pseudomonas putida et du Geotrichum candidum, le rapport du nombre de cellules étant de 4/1.

6. Mélange suivant la revendication 1, caractérisé en ce qu'il contient de la Pseudomonas putida et du Geotrichum candidum, le rapport du nombre de cellules étant de 5/1.

7. Procédé pour cultiver le mélange suivant une des revendications 1 à 6, caractérisé en ce que le mélange est induit par culture en présence d'acide oléique, avec apport d'oxygène et, le cas échéant, avec apport de substances stimulantes, en particulier d'azote et de phosphore ainsi que, le cas échéant, d'oligo-éléments.

8. Procédé pour l'application du mélange suivant une des revendications 1 à 6, caractérisé en ce qu'une ou plusieurs fois entre 1 gramme et 10 grammes de mélange par litre de la préparation d'application sont appliqués dans un dosage de la préparation d'application, qui est, suivant le degré de souillure du sol, de 5 à 20 litres par m³ de sol à traiter, et que de l'oxygène ainsi que des substances stimulantes, notamment de l'azote, du phosphore et des oligo-éléments, sont ajoutés en quantité optimale, le rapport entre le carbone et l'azote étant maintenu dans une plage de 10/1 à 100/1 pendant l'action du mélange.

9. Procédé suivant la revendication 8, caractérisé en ce que le milieu est maintenu à un pH compris entre 4,5 et 7,5 ainsi qu'à une température comprise entre 5° et 35°C.

10. Utilisation d'un mélange suivant une des revendications 1 à 6 pour la dégradation industrielle, artisanale ou commerciale d'hydrocarbures dans les sols et les eaux.

## Claims

1. Mixture of natural microorganisms for the biodegradation of hydrocarbons, in particular of crude oil and crude oil-products, characterized in that it contains Pseudomonas putida according to the international deposit No. CCM 4307 with the designation *Pseudomonas sp. RC1,* as well as Geotrichum candidum according to the international deposit No. CCM 8170 with the designation *Geotrichum candidum RC1* in a cell-number ratio of 5 : 1 to 1 : 1.

2. Mixture according to claim 1, characterized in that it contains Pseudomonas putida and Geotrichum candidum in a cell-number ratio of 1 : 1.

3. Mixture according to claim 1, characterized in that it contains Pseudomonas putida and Geotrichum candidum in a cell-number ratio of 2 : 1.

4. Mixture according to claim 1, characterized in that it contains Pseudomonas putida and Geotrichum candidum in a cell-number ratio of 3 : 1.

5. Mixture according to claim 1, characterized in that it contains Pseudomonas putida and Geotrichum candidum in a cell-number ratio of 4 : 1.

6. Mixture according to claim 1, characterized in that it contains Pseudomonas putida and Geotrichum candidum in a cell-number ratio of 5 : 1.

7. Process for the cultivation of the mixture according to on of the claims 1 to 6, characterized in that the mixuture is being induced by a cultivation in the presence of oil acid, with supply of oxygen and if necessary with supply of stimulation substances, in particular of nitrogen and phosphor, as well as, if necessary, trace elements.

8. Process for the application of the mixutre according to one of the claims 1 to 6, characterized in that one or several times between 1 gram and 10 grams of mixture per liter of application preparation in a dosage of the application preparation of, depending on the degree of the contamination, 5 to 20 liters per cubic meter soil to be treated is being applied, further that oxygen, phosphor as well as trace elements in optimal quantity are being supplied, whereby the ratio of the carbon to the nitrogen during the activity of the mixture is being kept in a range of 10 : 1 to 100 : 1.

9. Process according to claim 8, characterized in that the environment is being kept on a pH-value of 4.5 to 7.5 and on a temperature of 5° to 35°C.

10. Use of a mixture according to one of the claims 1 to 6 for the commercial degradation of hydrocarbons in soils and waters.
